(19)

Europäisches
Patentamt
European
Patent Office
Office européen
des brevets

(11)  EP 4 209 271 A1

(12)  EUROPEAN PATENT APPLICATION
published in accordance with Art. 153(4) EPC

(43) Date of publication:
12.07.2023  Bulletin 2023/28

(51) International Patent Classification (IPC):
B01J 35/10 $^{(2006.01)}$      F01N 3/08 $^{(2006.01)}$
B01J 23/34 $^{(2006.01)}$      B01J 23/68 $^{(2006.01)}$
A61L 9/00 $^{(2006.01)}$      A61L 9/01 $^{(2006.01)}$

(21) Application number: 21864398.9

(22) Date of filing: 02.09.2021

(52) Cooperative Patent Classification (CPC):
A61L 9/00; A61L 9/01; B01J 23/34; B01J 23/68;
B01J 35/10; F01N 3/08; Y02A 50/20

(86) International application number:
PCT/JP2021/032257

(87) International publication number:
WO 2022/050343 (10.03.2022 Gazette 2022/10)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority:  03.09.2020  JP 2020148138

(71) Applicants:
• NBC Meshtec Inc.
Hino-shi, Tokyo 191-0053 (JP)
• Tokyo Metropolitan Public University
Corporation
Tokyo 163-0926 (JP)
• The University of Tokyo
Bunkyo-ku, Tokyo 113-8654 (JP)

(72) Inventors:
• FUKUI, Yoko
Hino-shi, Tokyo 191-0053 (JP)
• JIKIHARA, Youhei
Hino-shi, Tokyo 191-0053 (JP)
• MURAYAMA, Toru
Hachioji-shi, Tokyo 192-0397 (JP)
• SHISHIDO, Tetsuya
Hachioji-shi, Tokyo 192-0397 (JP)
• TAKEI, Takashi
Hachioji-shi, Tokyo 192-0397 (JP)
• LIN, Mingyue
Hachioji-shi, Tokyo 192-0397 (JP)
• WANG, Haifeng
Hachioji-shi, Tokyo 192-0397 (JP)
• YAMAGUCHI, Kazuya
Tokyo 113-8654 (JP)

(74) Representative: J A Kemp LLP
80 Turnmill Street
London EC1M 5QU (GB)

(54)  **DEODORIZING CATALYST**

(57)  [Problem] The purpose of the present invention is to provide a deodorizing catalyst that can decompose a malodorous substance even at a low temperature of 100°C or less.

[Solution] A deodorizing catalyst for decomposing a malodorous substance, comprising: manganese oxide wherein the manganese oxide satisfies the following expression (1) and the following expression (2), and the manganese oxide has a maximum intensity peak at a diffraction angle (2Θ) of 37 $\pm$ 1° in an X-ray diffraction pattern:

$$0 < A \leq 0.90 \ ... \ (1)$$

$$0 < B \leq 250 \ ... \ (2)$$

wherein, in the above-mentioned expression (1), A represents the content ratio of manganese having an oxidation number of 3 ($Mn^{3+}$) to manganese having an oxidation number of 4 ($Mn^{4+}$) ($Mn^{3+}/Mn^{4+}$) in the manganese oxide, and, in the above-mentioned expression (2), B represents a specific surface area of the manganese oxide ($m^2/g$).

EP 4 209 271 A1

**Description**

Technical Field

The present invention relates to a deodorizing catalyst that can decompose a malodorous substance such as ammonia, amines, or sulfur compounds.

Background Art

When natural organic substances such as seafood and the vegetables and fruits putrefy, various malodorous substances are produced. A malodor such as smell of feces and urine is also produced from feces and urine. Nitrogen compounds such as ammonia and trimethylamine; sulfur compounds such as hydrogen sulfide and methyl mercaptan produced from sulfur-containing amino acids; and organic compounds such as formic acid, butyric acid, and acetic acid are known as malodorous substances. In places such as waste disposal facilities or toilets that may produce malodorous substances, various means for removing these are employed.

As methods for removing malodorous substances, the adsorption of malodorous substances on adsorbents such as activated carbon, methods for decomposing and removing malodorous substances with active species such as radicals and ozone generated with plasma generating devices are widely used. In the case of the absorptive treatments with adsorbents, the absorbed amounts however have upper limits, and the adsorbents need to be periodically replaced. The decomposition with the plasma or the like is complicated methods that need generating devices and electric power for operating the devices.

Methods for promoting the oxidation reaction using various catalysts for decomposition have been examined besides methods for oxidation and decomposition with active species physically generated such as the above-mentioned plasma methods. For example, a catalyst containing a platinum-group element and a metal oxide having an acid strength of -5.6 or more (Patent Literature 1) and the like have been examined.

Manganese compounds have attracted attention as low-price catalysts among catalysts for promoting oxidation reaction. For example, in Patent Literature 2, an ammonia-decomposing catalyst which is mixture of a zeolite having an $SiO_2/Al_2O_3$ molar ratio of 10 or more and manganese oxide are mixed has been examined.

Citation List

Patent Literature

  Patent Literature 1 JP2009-254981A
  Patent Literature 2 JP2007-216082A

Summary of Invention

Technical Problem

It is desirable that reaction for decomposing a malodorous substance proceed even at a low temperature of 100°C or less. For example, a catalyst needs to decompose a malodorous substance at a temperature for storing food, namely at a temperature of room temperature or less, in order to decompose a malodorous substance produced from food.

Here, since the catalyst of Patent Literature 1 is a catalyst containing a platinum-group element, the production cost is high, and the uses thereof are limited. The catalyst of Patent Literature 2 oxidizes (decomposes) ammonia at a very high decomposition reaction temperature of 350°C, and ammonia decomposition at low temperature has not been examined at all.

An object of the present invention is to provide a deodorizing catalyst that can decompose a malodorous substance even at a low temperature of 100°C or less.

Solution to Problem

The present inventors have found that manganese oxide that satisfies specific conditions can decompose a malodorous substance such as ammonia, an amines, or sulfur compounds, especially ammonia, amines, or the like at a low temperature of 100°C or less and completed the present invention.

The subject matter of the present invention is as follows.

[1] A deodorizing catalyst for decomposing a malodorous substance, comprising: manganese oxide wherein the manganese oxide satisfies the following expression (1) and the following expression (2) and the following expression (2), and the manganese oxide has a maximum intensity peak at a diffraction angle ($2\Theta$) of $37 \pm 1°$ in an X-ray diffraction pattern:

$$0 < A \leq 0.90 \ldots (1)$$

$$0 < B \leq 250 \ldots (2)$$

wherein, in the above-mentioned expression (1), A represents the content ratio of manganese having an oxidation number of 3 ($Mn^{3+}$) to manganese having an oxidation number of 4 ($Mn^{4+}$) ($Mn^{3+}/Mn^{4+}$) in the manganese oxide, and, in the above-mentioned expression (2), B represents the specific surface area of the manganese oxide ($m^2/g$).

[2] The deodorizing catalyst according to [1], wherein the deodorizing catalyst further satisfies the following expression (3):

$$0 < A \cdot B \leq 200 \ldots (3)$$

wherein, in the above-mentioned expression (3), A and B are synonymous with the above.

[3] The deodorizing catalyst according to [1] or [2], wherein the manganese oxide comprises cations of an alkali metal element.

[4] The deodorizing catalyst according to any one of [1] to [3], wherein metal particles are carried on the manganese oxide, and a metal component comprised in the metal particles is one or more selected from the group consisting of gold, platinum, silver, copper, palladium, rhodium, iridium, ruthenium, osmium, and rhenium.

[5] The deodorizing catalyst according to any one of [1] to [4], wherein the malodorous substance is one or more substances selected from the group consisting of ammonia, amines, and sulfur compounds.

[6] A deodorizing body, comprising: the deodorizing catalyst according to any one of [1] to [5] and an adsorbent.

[7] A deodorant method, wherein the deodorizing catalyst according to any one of [1] to [5] is contacted with fluid to be treated comprising the malodorous substance in the presence of oxygen at a temperature of 20°C or more and 100°C and less.

[8] The deodorant method according to [7], wherein the deodorizing catalyst is contacted with the fluid to be treated in an environment of a relative humidity of 50% or more.

Advantageous Effects of Invention

According to the present invention, a deodorizing catalyst that can decompose a malodorous substance even at a low temperature of 100°C or less can be provided.

Description of Embodiments

Hereinafter, embodiments of the present invention will be described in detail.

A deodorizing catalyst for a malodorous substance of the present embodiment can decompose gas to be treated or liquid to be treated comprising a malodorous substance (hereinafter, these are also referred to as "fluid to be treated") by contacting the fluid to be treated with the deodorizing catalyst in the presence of oxygen so as to decompose the malodorous substance in the liquid to be treated. Here, the decomposition reaction of the malodorous substance with the deodorizing catalyst of the present embodiment is, for example, an oxidative decomposition reaction in which the malodorous substance is oxidized and decomposed.

The malodorous substance to be decomposed is a substance having an unpleasant smell, and examples thereof include volatile organic compounds, nitrogen compounds, and sulfur compounds. Examples of the volatile organic compounds include alcohols such as butanol; aldehydes such as acetaldehyde; and fatty acids such as valeric acid. Examples of the nitrogen compounds include ammonia, amines such as trimethylamine, and examples of the sulfur compounds include hydrogen sulfide and thiols such as methanethiol. The deodorizing catalyst of the present embodiment is particularly effective in the oxidative decomposition of sulfur compounds, ammonia, and amines.

When ammonia or amines are subjected to oxidative decomposition reaction, nitrogen contained in the ammonia and the amines is usually oxidatively decomposed into nitrogen gas or nitrogen oxide. Therefore, it is insufficient for the deodorizing catalyst to merely decompose the malodorous substance, and it is necessary to be able to promote the oxidation reaction for producing safe substances without by-producing a harmful substance such as carbon monoxide or nitrogen oxide. Examples of the harmful substance include carbon monoxide, which can be by-produced by decomposing fatty acids, and hydrogen sulfide, which can be by-produced by decomposing a sulfur compound, besides nitrogen oxide, described above.

The deodorizing catalyst of the present embodiment can decompose the malodorous substance even at a low temperature of 100°C or less, and tends to hardly produce the harmful substance in addition even though the malodorous substance is decomposed. Especially when the deodorizing catalyst of the present embodiment satisfies the expression (4), described below, besides the expression (1) and the expression (2), described below, a nitrogen element contained in ammonia or amines is easily converted into nitrogen gas, and harmful substances such as nitrogen oxide are hardly produced in the process in which ammonia or amines are decomposed. That is, one aspect of the deodorizing catalyst of the present embodiment also has the characteristic of having a high nitrogen selectivity.

The nitrogen selectivity used herein is defined as the rate of nitrogen that changes into substances other than nitrogen monoxide, nitrogen dioxide, and dinitrogen monoxide among nitrogen (N) contained in ammonia or amines decomposed with the deodorizing catalyst. A specific calculation expression is shown in the following expression (5).

$$\text{Nitrogen selectivity (\%)} = [\{(CAa - CAb) - (CN1b + CN2b + C2Nb)\}/(CAa - CAb)]*100 \ldots (5)$$

CAa: Nitrogen amount of ammonia and/or amines comprised in gas to be treated before feeding to deodorizing catalyst (mol/L)
CAb: Nitrogen amount of ammonia and/or amines comprised in gas to be treated after feeding to deodorizing catalyst (mol/L)
CN1b: Nitrogen amount of nitrogen monoxide comprised in gas to be treated after feeding to deodorizing catalyst (mol/L)
CN2b: Nitrogen amount of nitrogen dioxide comprised in gas to be treated after feeding to deodorizing catalyst (mol/L)
C2Nb: Nitrogen amount of dinitrogen monoxide comprised in gas to be treated after feeding to deodorizing catalyst (mol/L)

The deodorizing catalyst of the present embodiment comprises manganese oxide that satisfies the following expression (1) and the following expression (2), and has a maximum intensity peak at a diffraction angle (2Θ) of 37 ± 1° in an X-ray diffraction pattern.

$$0 < A \leq 0.90 \ldots (1)$$

$$0 < B \leq 250 \ldots (2)$$

wherein, in the above-mentioned expression (1), A represents the content ratio of manganese having an oxidation number of 3 ($Mn^{3+}$) to manganese having an oxidation number of 4 ($Mn^{4+}$) ($Mn^{3+}/Mn^{4+}$) in the manganese oxide, and, in the above-mentioned expression (2), B represents the specific surface area of the manganese oxide ($m^2/g$) .

When the X-ray diffraction was measured, as described above, the manganese oxide comprised in the deodorizing catalyst has a maximum intensity peak at a diffraction angle (2Θ) of 37 ± 1° in the X-ray diffraction pattern. Here, the maximum intensity peak in the X-ray diffraction pattern refers to the peak having the highest intensity in an X-ray diffraction pattern obtained by measuring X-ray diffraction under the conditions described in the Examples, mentioned below. Also, the maximum intensity peak at a diffraction angle (2Θ) of 37 ± 1° refers to said maximum intensity peak whose peak top appears in the diffraction angle (2Θ) range of 37 ± 1°.

As long as the manganese oxide comprised in the deodorizing catalyst has the maximum intensity peak at the diffraction angle (2Θ) of 37 ± 1°, the manganese oxide may not be a single crystal, and amorphous portions may coexist therein.

It is known that, in manganese oxide, manganese having oxidation numbers of 2 (+2), 3 (+3), 4 (+4), 6 (+6), and 7 (+7) exists. Not only manganese oxide comprises manganese having a single oxidation number but also manganese oxide in which manganese having multiple oxidation numbers coexists are known. In the manganese oxide according to the present embodiment, the content ratio of manganese having an oxidation number of 3 (hereinafter also referred to merely as "$Mn^{3+}$") to manganese having an oxidation number of 4 (hereinafter also referred to merely as "$Mn^{4+}$") (hereinafter also referred to merely as "$Mn^{3+}/Mn^{4+}$") is more than 0 and 0.90 or less (that is, the manganese oxide satisfies the above-mentioned expression (1)).

Although the reason why the deodorizing catalyst of the present embodiment can decompose the malodorous substance even at a low temperature of 100°C or less is not appreciated, it is believed that local distortions occur in the crystal structure, and the distortions are active spots as the catalyst in the case where manganese of $Mn^{3+}$ and $Mn^{4+}$ coexists as compared with manganese oxide comprising manganese having a single oxidation number. Therefore, it is inferred that when the manganese oxide satisfies the above-mentioned expression (1), active spots are easily generated as compared with when the $Mn^{3+}/Mn^{4+}$ is 0 (A = 0) and when the $Mn^{3+}/Mn^{4+}$ is more than 0.9 (0.90 < A). The $Mn^{3+}/Mn^{4+}$ is preferably 0.20 or more and 0.90 or less ($0.20 \leq A \leq 0.90$), and more preferably 0.30 or more and 0.90 or less ($0.30 \leq A \leq 0.90$) from the viewpoint of decomposing the malodorous substance more easily.

The manganese oxide comprised in the deodorizing catalyst satisfies the condition that A, which represents $Mn^{3+}/Mn^{4+}$, is more than 0 and 0.90 or less (the above-mentioned expression (1)), and further satisfies the condition that B, which represents the specific surface area of the manganese oxide ($m^2/g$), is more than 0 and 250 or less (the above-mentioned expression (2))in addition. Although the relationship between the satisfaction of the above-mentioned expression (2) and the tendency to hardly by-produce a harmful substance is not appreciated, it is inferred that the density of the active spots described above is not too excessive, excessive oxidation of the malodorous substance (for example, ammonia) hardly proceeds, and the harmful substance tends to be hardly by-produced (for example, the nitrogen selectivity increases). The specific surface area of the manganese oxide (B) is preferably 30 $m^2/g$ or more and 250 $m^2/g$ or less, and more preferably 40 $m^2/g$ or more and 250 $m^2/g$ or less from the viewpoint of further facilitating the decomposition of the malodorous substance or further preventing the harmful substance from being by-produced (the nitrogen selectivity further increases). When the metal particles described below is carried on the manganese oxide, the specific surface area (B) of the manganese oxide refers to the specific surface area of the manganese oxide including the metal particles (the specific surface area considering the surface area of the metal particles).

Although the manganese oxide comprised in the deodorizing catalyst is not particularly limited, it is preferable that the manganese oxide satisfy the above-mentioned expression (1) and the above-mentioned expression (2), and further satisfy the following expression (3) additionally. It is more preferable that the manganese oxide satisfy the above-mentioned expression (1) and the above-mentioned expression (2), and further satisfy the following expression (4) additionally. When the manganese oxide satisfies the following expression (3), the manganese oxide decomposes the malodorous substance even at low temperature more easily. When the manganese oxide satisfies the following expression (4), the manganese oxide decomposes the malodorous substance more easily, and more hardly by-produces the harmful substance in addition (the nitrogen selectivity further increases).

$$0 < A \cdot B \leq 200 \quad \ldots \quad (3)$$

$$0 < A \cdot B \leq 100 \quad \ldots \quad (4)$$

wherein, in the above-mentioned expression (3) and the above-mentioned expression (4), A and B are synonymous with the above.

The ratio of $Mn^{3+}$ to $Mn^{4+}$ ($Mn^{3+}/Mn^{4+}$) can be calculated using X-ray photoelectron spectroscopy described in the Examples, mentioned below. The nitrogen adsorption measurement at the temperature of liquid nitrogen (-196°C) can be performed to calculate the specific surface area of the manganese oxide using an automatic specific surface area measuring device by the BET method.

In the deodorizing catalyst of the present embodiment, cations of a metallic element that is different from manganese may be further comprised in the manganese oxide. Examples of the type of the metallic element other than manganese that can be comprised in the manganese oxide include, but are not particularly limited to, alkali metal elements such as Li, Na, and K and transition metal elements such as Mg, Ca, St, Ba, Zn, Cu, Ni, Co, and Fe. The alkali metal elements and the transition metal elements are desirable due to an increase in the stability of the deodorizing catalyst, or the like. Although the content of the metallic element (cations) that is different from manganese is not particularly limited, it is desirable that the content be 0.01% by mass or more and 20% by mass or less based on 100% by mass of the deodorizing catalyst.

Next, a method for producing the deodorizing catalyst of the present embodiment will be described. In the following description, the manganese oxide that satisfies the above-mentioned expression (1) and the above-mentioned expression (2) and which has the maximum intensity peak at the diffraction angle ($2\Theta$) of $37 \pm 1°$ in the X-ray diffraction pattern is called the "manganese oxide that satisfies the predetermined conditions".

The method for producing the deodorizing catalyst of the present embodiment comprises an acquisition step of acquiring manganese oxide and a baking step of baking the manganese oxide obtained in the acquisition step. Although various methods such as the solid phase method, the molten salt flux method, and the oxidation-reduction precipitation method are known as the method for acquiring manganese oxide, it is preferable to use the oxidation-reduction precipitation method in the acquisition step described above. Since the reaction conditions are mild, and a complicated production device is not required, the oxidation-reduction precipitation method is desirable.

In the case where the oxidation-reduction precipitation method is used, manganese oxide is acquired as a precipitate by reacting a reducer with a manganese compound the oxidation number of which is larger than 4 when dissolved in an aqueous solution or reacting an oxidizer with a manganese compound the oxidation number of which is smaller than 3 when dissolved in an aqueous solution. It is preferable that the oxidizer or the reducer be a manganese compound in the oxidation-reduction precipitation method described above. When the manganese compound is used as the oxidizer or the reducer, the manganese oxide of the present embodiment having a small amount of impurities (substances other than manganese and oxygen) is obtained, and the manganese compound is therefore preferable. It is particularly preferable that a manganese compound having an oxidation number of 2 (hereinafter also referred to as a "divalent manganese compound") and a manganese compound having an oxidation number of 7 (hereinafter also referred to as a "heptavalent manganese compound") be reacted to reduce the heptavalent manganese compound with the divalent manganese compound and oxidize the divalent manganese compound with the heptavalent manganese compound. When the oxidation-reduction precipitation method in which the divalent manganese compound and the heptavalent manganese compound are reacted is used, manganese oxide which has the maximum intensity peak at the diffraction angle ($2\Theta$) of $37 \pm 1°$ in the X-ray diffraction pattern is easily obtained as compared with oxidation-reduction precipitation methods in which other substances that are different from these are reacted, and the oxidation-reduction precipitation method is therefore particularly preferable.

As the heptavalent manganese compound, for example, potassium permanganate ($KMnO_4$) can be used, and as the divalent manganese compound, for example, manganese sulfate (II) ($MnSO_4$) can be used. A permanganate other than $KMnO_4$ can also be used as the heptavalent manganese compound. For example, $NaMnO_4 \cdot 3H_2O$, $AgMnO_4$, $Zn(MnO_4)_2 \cdot 6H_2O$, $Mg(MnO_4)_2$, $Ca(MnO_4)_2$, $Ba(MnO_4)_2$, or the like can be used. As the divalent manganese compound, a manganese salt other than $MnSO_4$ having an oxidation number of 2 (hereinafter also referred to as a "divalent manganese salt") can also be used. For example, manganese chloride, manganese carbonate, manganese nitrate, or the like can be used.

When the oxidation-reduction precipitation method in which the divalent manganese compound and the heptavalent manganese compound are reacted is used, as one example, the following reaction conditions are used. When manganese oxide obtained under the following reaction conditions is subjected to baking treatment described below, manganese oxide that satisfies the predetermined conditions can be obtained. The heptavalent manganese compound (permanganate) and the divalent manganese compound (divalent manganese salt) are reacted in an aqueous solution at a reaction temperature of 20 to 200°C for 0.5 hours to 36 hours using a pressure container as needed. The molar ratio of the heptavalent manganese compound (permanganate) to be reacted to the divalent manganese compound (manganese salt) to be reacted is 0.05 or more and 10 or less, and it is preferable that the ratio be 0.5 or more and 10 or less to increase the yield. When the divalent manganese compound and the heptavalent manganese compound are reacted in an acidic aqueous solution, manganese oxide which has the maximum intensity peak at the diffraction angle ($2\Theta$) of $37 \pm 1°$ in the X-ray diffraction pattern is more easily obtained, and the reaction in the acidic aqueous solution is desirable. In the case of the above-mentioned method for producing manganese oxide, the aqueous solution is usually an acidic aqueous solution, but the pH of the aqueous solution may be adjusted to the acidic condition by adding an acid separately as needed for reaction. Although the type of the acid to be added is not particularly limited, the amount of acid added can be reduced in the cases of an inorganic strong acid such as hydrochloric acid, nitric acid, or sulfuric acid. The inorganic strong acid is therefore preferable.

Although the reaction conditions such as the reaction temperature in the oxidation-reduction precipitation method influence the $Mn^{3+}/Mn^{4+}$ (A in the above-mentioned expression (1)), the specific surface area (B in the above-mentioned expression (2)), the X-ray diffraction pattern, or the like of the obtained manganese oxide, it is difficult to obtain the manganese oxide that satisfies the predetermined conditions only by changing the reaction conditions. In other words, with respect to the manganese oxide obtained in the acquisition step, the $Mn^{3+}/Mn^{4+}$ exceeds 0.90 (that is, the above-mentioned expression (1) is not satisfied), or the specific surface area of the manganese oxide exceeds 250 ($m^2/g$) (that is, the above-mentioned expression (2) is not satisfied). Accordingly, the manganese oxide obtained in the acquisition step is baked to obtain the manganese oxide that satisfies the predetermined conditions.

When the manganese oxide obtained in the acquisition step is subjected to baking treatment, the $Mn^{3+}/Mn^{4+}$ can be further reduced as compared with the manganese oxide obtained in the acquisition step (the manganese oxide before the baking). When the manganese oxide obtained in the acquisition step is subjected to the baking treatment, the specific surface area of the manganese oxide can be further reduced as compared with the manganese oxide obtained in the acquisition step (the manganese oxide before the baking).

In the baking step, the baking temperature varies depending on the $Mn^{3+}/Mn^{4+}$ and the specific surface area of the manganese oxide before the baking, for example, the baking temperature can be adjusted to the temperature range of 100°C or more and 700°C or less. The baking time varies depending on the $Mn^{3+}/Mn^{4+}$ and the specific surface area of the manganese oxide to be baked, for example, the baking time can be adjusted to 1 to 24 hours. When the baking temperature is increased, the specific surface area of the manganese oxide tends to further decrease. When the baking time is extended, the value of the $Mn^{3+}/Mn^{4+}$ tends to decrease. Therefore, the baking temperature and the baking time only have to be adjusted in view of these tendencies so that the above-mentioned expression (1) and the above-mentioned expression (2) are satisfied.

The manganese oxide obtained in the baking step described above can be used as the deodorizing catalyst of the present embodiment. The method for producing the deodorizing catalyst may comprise a filtering step of filtering the manganese oxide produced by the reaction in the acquisition step, a washing step of washing the filtered manganese oxide with water, and a drying step of drying the washed manganese oxide in addition to the acquisition step and the baking step described above. Well-known conditions can be used as the conditions for the filtering, the washing, or the drying, and the conditions are not particularly limited.

Although the shape of the manganese oxide (the shape of the deodorizing catalyst) is not particularly limited, for example, the shape can be a powdery shape. The manganese oxide (deodorizing catalyst) may be immobilized on a substrate, or may be molded into a filter shape or a pellet shape depending on the use. The manganese oxide (deodorizing catalyst) may be also a powdery shape having pores.

When the shape of the manganese oxide (deodorizing catalyst) is a powdery shape, for example, the average particle size thereof can be 0.005 to 100 $\mu$m, and the average particle size can moreover be 0.1 to 10 $\mu$m. The particle size can be adjusted by a pulverization method well-known to those skilled in the art such as a ball mill.

The deodorizing catalyst of the present embodiment may be used as a deodorizing body that is a composition with an adsorbent. The deodorizing catalyst of the present embodiment can be used even at low temperature (100°C or less) as compared with conventional deodorizing catalysts, the deodorizing effect can be further enhanced in combination with the adsorbent. Even though the adsorption sites of the adsorbent are saturated by the adsorption of the malodorous substance, the deodorizing body can be recycled and used repeatedly by heating the deodorizing body. Although the malodorous substance is released from the adsorbent by heating, the malodorous substance is decomposed with the manganese oxide that exists in the deodorizing body.

Although the adsorbent is not particularly limited, a substance such as activated carbon, silica gel, zeolite, or alumina well-known to those skilled in the art can be used.

In order that the catalyst is hardly influenced by water, the catalyst may be made hydrophobic by a well-known method, or a moisture absorbent or a drying agent may be added.

In the deodorizing catalyst of the present embodiment, metal particles may be further carried on the manganese oxide. When the metal particles are carried, the malodorous substance is more easily decomposed, or the harmful substance is more hardly by-produced. Additionally, when a metal particle is carried, the minimum decomposition temperature of the malodorous substance can be further reduced. A metal component comprised in the metal particles may be a zerovalent simple substance (that is, a metal state) or an oxide, or may be in a state in which a zerovalent simple substance and an oxide coexist. Two or more metallic elements may be used for the metal component. In that case, the two or more metallic elements may exist as an alloy, or may exist as a metal mixture.

As the metal component comprised in the metal particles, gold, platinum, silver, copper, palladium, rhodium, iridium, ruthenium, osmium, and rhenium are exemplified, but silver or copper is low-priced and easily handled in the production, and silver or copper is therefore preferable.

Although the method for make the metal particles carried is not particularly limited, the metal particles can be carried by a method, such as a method for dispersing (immersing) the manganese oxide in a solution of the metal salt and adsorbing the metal salt on the surface of the manganese oxide for baking in the presence of a reducer as necessary, well-known to those skilled in the art. When the metal particle is carried, the specific surface area (B) of the manganese oxide tends to increase, the specific surface area (B) may therefore be adjusted by baking treatment for carry the metal particles so that the specific surface area (B) does not exceed 250 m$^2$/g. As described above, when the baking temperature is increased, the specific surface area of the manganese oxide tends to further decrease, and when the baking time is extended, the value of $Mn^{3+}/Mn^{4+}$ tends to decrease. The baking treatment to be performed for making the metal particles carried can be therefore performed under the conditions that the manganese oxide carrying a metal particle after the baking satisfies the above-mentioned expression (1) and the above-mentioned expression (2) in view of these tendencies.

Since the catalytic activity can be further enhanced, the average particle size of the metal particles is preferably 10 nm or less, and particularly preferably 0.1 to 10 nm.

When the particle size used herein is the size of a particle that can be measured from an image photograph through a transmission electron microscope (TEM), and the average particle size used herein is a value obtained by averaging the sizes of target particles, and is a value obtained by averaging the sizes of at least 200 particles in the case where the number of the target particles exceed 200.

Since the catalytic activity can be further enhanced, it is preferable that the amount of the metal particles according to the present embodiment carried be 0.1 to 15.0% by mass, and it is further preferable that the amount be 0.5 to 12.0% by mass based on 100% by mass of the manganese oxide (the manganese oxide that does not carry the metal particles).

Next, a method for decomposing the malodorous substance with the deodorizing catalyst of the present embodiment will be described. As described above, the deodorizing catalyst of the present embodiment can decompose the malodorous substance in the fluid to be treated by contacting the fluid to be treated comprising the malodorous substance with the deodorizing catalyst in the presence of oxygen.

Although the concentration of the malodorous substance in the fluid to be treated is not particularly limited, it is preferable that the concentration be 5 ppm by volume or more and 50% by volume or less in the case of gas, and the concentration be 5 ppm by volume or more and 50% by weight or less in the case of liquid based on the fluid to be treated from the viewpoint of enabling an increase in the decomposition rate. Other components than the malodorous substance (oxygen as necessary) may be comprised in the fluid to be treated.

Although the method and the conditions for contacting the fluid to be treated with the deodorizing catalyst are not particularly limited, it is preferable that the fluid to be treated be contacted with the deodorizing catalyst at a space velocity of 1, 000 mlh$^{-1}$g-cat$^{-1}$ or more and 7,000,000 mlh$^{-1}$g-cat$^{-1}$ or less from the viewpoint that the harmful substance is hardly by-produced (the nitrogen selectivity can be further increased). The space velocity represents the ratio of the volume of fluid to be treated that is contacted with (passes) a deodorizing catalyst per unit time to the volume or the weight of the deodorizing catalyst as a velocity. The space velocity is a value obtained by dividing the flow rate (volume) of the fluid to be treated per unit time by the volume or the weight of the deodorizing catalyst. The fluid to be treated may be contacted with the deodorizing catalyst under the atmospheric pressure or in a reduced pressure atmosphere or a pressurized atmosphere.

Examples of the method for contacting the fluid to be treated with the deodorizing catalyst in the presence of oxygen include a method for contacting fluid to be treated comprising oxygen with a deodorizing catalyst and a method for contacting fluid to be treated and gas comprising oxygen with a deodorizing catalyst. The oxygen concentration is not particularly limited, and the oxygen concentration can be equivalent to the oxygen concentration of the atmosphere as the gas comprising oxygen. However, the oxygen concentration is preferably 1.0 time or more the concentration of the malodorous substance (for example, ammonia, amines, sulfur compounds such as hydrogen sulfide) comprised in the fluid to be treated based on volume from the viewpoint of increasing the decomposition rate of the malodorous substance.

Although the temperature for contacting the fluid to be treated with the deodorizing catalyst is not particularly limited, the temperature is preferably 20°C or more and 100°C or less, and more preferably 50°C or more and 100°C or less. Conventional decomposition methods have a problem that when the fluid to be treated is not contacted with the catalyst at a high temperature of more than 100°C, the malodorous substance is hardly decomposed, and nitrogen oxide is easily produced due to high temperature. Even though the fluid to be treated is contacted with the catalyst at a low temperature of 100°C or less, the malodorous substance can however be decomposed in the decomposition method of the present embodiment. Furthermore, the decomposition method tends to enable suppressing the production of a harmful substance such as nitrogen oxide (that is, the nitrogen selectivity tends to be high). The fluid to be treated may be contacted with the deodorizing catalyst at a temperature of more than 100°C. In the case of the contact at a temperature of more than 100°C, the malodorous substance is more easily decomposed as compared with when the fluid to be treated is contacted with the deodorizing catalyst at 100°C or less.

The temperature for contacting the fluid to be treated with the deodorizing catalyst can be adjusted by heating or cooling the deodorizing catalyst, and the temperature can also be adjusted by heating or cooling the fluid to be treated. The temperature may be adjusted by heating or cooling both the deodorizing catalyst and the fluid to be treated. For example, the fluid to be treated can be contacted with the deodorizing catalyst at a temperature of 20°C or more and 100°C or less by adjusting the temperature of the deodorizing catalyst to 20°C or more and 100°C or less or by adjusting the temperature of the fluid to be treated to 20°C or more and 100°C or less.

Although the humidity environment when the deodorizing catalyst of the present embodiment is used is not particularly limited, the deodorizing catalyst can be appropriately used even in a high humidity environment of a relative humidity of 50% or more. Since the catalytic activity in a high humidity environment decreases rapidly, conventional deodorizing catalysts are difficultly used in a high humidity environment, and the drying treatment of the gas to be treated or the like is required beforehand. When the deodorizing catalyst of the present embodiment is used, the malodorous substance in a high humidity environment can however be decomposed even without such advance drying treatment. It is inferred that although, in the conventional deodorizing catalyst, the catalytic activity may decrease due to the adhesion of water produced by the oxidation reaction to the catalyst, as described above, the deodorizing catalyst of the present embodiment has high resistance to humidity, the decomposition of the malodorous substance can be promoted even at low temperature.

The deodorizing catalyst of the present embodiment can be used, for example, for a member or a gas purifier having the action or the function of removing ammonia, amines, or sulfur compounds such as hydrogen sulfide, and can be used by contacting the gas to be treated comprising ammonia or amines with the catalyst in the presence of oxygen. Examples of the member and the device include deodorizing devices to be installed in air conditioners, refrigerators, warehouses, and showcases; deodorizers for stock-raising sites; air purifiers to be installed in offices, smoking rooms, toilets, hospitals, nursing homes, and the like; and exhaust gas purifiers for internal combustion engines and the like.

The members or the devices may be provided with functions such as heating mechanisms for the deodorizing catalyst and ventilation mechanisms for sending the gas to be treated to the catalyst that enable setting use environments in which the deodorizing catalyst functions appropriately. The deodorizing catalyst of the present embodiment can also be used as a malodor suppressant for suppressing a malodor. As long as the malodor suppressant enables the contact of the gas to be treated comprising the malodorous substance with the catalyst in the presence of oxygen, the dosage form is not particularly limited, other components than the deodorizing catalyst may be comprised.

Examples

Next, the present invention will be described more specifically by giving the Examples. However, the present invention is not limited to only these Examples.

"Example 1"

First, 5.85 g (37 mmol) of $KMnO_4$ was dissolved in 100 mL of pure water. Then, 8.8 g (5.2 mmol) of $MnSO_4 \cdot H_2O$ was dissolved in 30 mL of pure water. Furthermore, 3 mL of nitric acid was added. Both solutions were mixed, and reflux reaction was performed at 100°C for 24 hours. The produced solid content was filtered, washed with pure water several times and then baked at 120°C for 6 hours to obtain the deodorizing catalyst (manganese oxide) of Example 1.

"Example 2"

First, 1.25 g (7.9 mmol) of $KMnO_4$ was dissolved in 40 mL of pure water. Then, 0.525 g (3.1 mmol) of $MnSO_4 \cdot H_2O$ was dissolved in 20 mL of pure water. Both solutions were mixed, and the mixture was stirred at room temperature (25°C) for 30 minutes. The produced solid content was filtered, washed with pure water several times and then dried at 60°C. Then, the dried solid content was baked at 150°C for 6 hours to obtain the deodorizing catalyst (manganese oxide) of Example 2.

"Example 3"

The same operation as in Example 1 was performed except that the reaction conditions of the reflux reaction in Example 1 were set at 80°C and 30 minutes to obtain the deodorizing catalyst (manganese oxide) of Example 3.

"Example 4"

First, $MnSO_4 \cdot H_2O$ and $KMnO_4$ were mixed in 75 mL of pure water so that the concentrations of $MnSO_4 \cdot H_2O$ and $KMnO_4$ are 0.196 mol/L and 0.14 mol/L, respectively, and the mixture was stirred at room temperature (25°C) for 30 minutes, then poured into a pressure container and heated at 160°C for 24 hours. The produced solid content was filtered, washed with pure water several times, then dried at 120°C and then fired at 500°C for 6 hours to obtain manganese oxide. The manganese oxide was subjected to the following silver-carrying step.

An aqueous ammonia solution was dropped into an aqueous silver nitrate solution to prepare an aqueous ammoniacal silver nitrate solution. The manganese oxide was added to pure water, the mixture was stirred for 30 minutes under ice-cooling and further stirred for 3 hours while the aqueous ammoniacal silver nitrate solution and hydrogen peroxide water were dropped. Then, the mixture was filtered and the solid content was collected. The solid content was washed with pure water several times, dried at 80°C and then fired at 400°C for 6 hours to obtain the deodorizing catalyst of Example 4 (silver-carried manganese oxide). The silver content was calculated from the difference between the masses of the manganese oxide before and after the silver-carrying step, and was 10% by mass based on 100% by mass of the manganese oxide. The average particle size was 2.7 nm from TEM observation.

"Example 5"

First, 2.2 g (13.9 mmol) of $KMnO_4$ was dissolved in 97.8 g of pure water to prepare an aqueous 2.2% by weight potassium permanganate solution. Then, 33.3 g of $MnSO_4 \cdot H_2O$ (197 mmol) was dissolved in 66.7 g of pure water to prepare an aqueous 33.3% by weight manganese sulfate solution. While both solutions were mixed and stirred with the liquid temperature adjusted to 20°C, the mixture was reacted at 20°C for 4 hours. The produced solid content was filtered and washed with pure water several times. The temperature was then raised to 300°C at a rate of temperature increase of 5°C per minute, and the washed solid content was successively baked at 300°C for 6 hours to obtain the deodorizing catalyst (manganese oxide) of Example 5.

"Example 6"

The same operation was performed as in Example 5 except that the baking condition in Example 5 was changed into 400°C and 6 hours, and the deodorizing catalyst of Example 6 was obtained.

"Example 7"

The manganese oxide obtained in Example 4 (manganese oxide before the silver-carrying step) was subjected to the following copper-carrying process. The manganese oxide was added to an aqueous 8.3 mM $CuCl_2$ solution, and the mixture was stirred at room temperature for 15 minutes. Then, the pH of the reaction solution was adjusted to 12 using an aqueous 1.0 M NaOH solution, and the reaction solution was successively stirred at room temperature for 24 hours. The solid content was collected by filtering from the reaction solution, and the solid content was washed with a large amount of pure water several times and then baked at 150°C for 12 hours to obtain the deodorizing catalyst of Example 7 (copper-carried manganese oxide). The copper content was calculated from the difference between the masses of the manganese oxide before and after the copper-carrying process, and was 5% by mass based on 100% by mass of the manganese oxide. The average particle size was 8.0 nm from TEM observation.

"Comparative Example 1"

Manganese oxide that was a commercial item (CMD-200, produced by Nippon Denko Co., Ltd.) was used as the deodorizing catalyst of Comparative Example 1 as it is.

"Comparative Example 2"

Manganese oxide that was a commercial item (AMD-200, produced by Japan Metals & Chemicals Co., Ltd.) was used as the deodorizing catalyst of Comparative Example 2 as it is.

"Comparative Example 3"

Manganese oxide that was a commercial item (AMD-250, produced by Japan Metals & Chemicals Co., Ltd.) was used as the deodorizing catalyst of Comparative Example 3 as it is.

"Comparative Example 4"

First, 2.0 g of potassium permanganate ($KMnO_4$) and 0.8 g of 95% sulfuric acid were dissolved in 100 mL of pure water to prepare a solution 1. Next, 2 mL of hydrogen peroxide water at a concentration of 30% was diluted with 100 mL of pure water to prepare a solution 2. Subsequently, the solution 2 was dropped with stirring the solution 1 at room temperature, and the mixture was further stirred for 30 minutes and left to stand for 12 hours. Then, the reaction solution was suction-filtered, the precipitate was obtained by further washing with pure water, and the obtained precipitate was collected, heated at 110°C for 12 hours and baked at 400°C for 2 hours to obtain the deodorizing catalyst (manganese oxide) of Comparative Example 4.

"Comparative Example 5"

First, 2.2 g of $KMnO_4$ was dissolved in 97.8 g of pure water to prepare an aqueous 2.2% by weight potassium permanganate solution. Then, 33.3 g of $MnSO_4 \cdot H_2O$ was dissolved in 66.7 g of pure water to prepare an aqueous 33.3% by weight manganese sulfate water solution. The liquid temperature was adjusted to 80°C, both solutions were mixed, and the mixture was reacted with stirring at 80°C for 4 hours. The product was filtered, washed with water several times and dried at 40°C for 48 hours to obtain the deodorizing catalyst (manganese oxide) of Comparative Example 5. The deodorizing catalyst of Comparative Example 5 was a solid having adhesiveness, and was difficultly handled. Even though the deodorizing catalyst of Comparative Example 5 was further dried at 40°C for 72 hours, the adhesiveness thereof did not disappear.

(Measurement of $Mn^{3+}/Mn^{4+}$)

Peaks attributed to $Mn^{3+}$ (hereinafter, referred to as a "$Mn^{3+}$ peak") and peaks attributed to $Mn^{4+}$ (hereinafter,

referred to as a "$Mn^{4+}$ peak") were subjected to waveform separation from XPS spectra obtained using an X-ray photoelectron spectroscope (XPS, manufactured by JEOL Ltd.: JPS-9010MX) . The values of the $Mn^{3+}/Mn^{4+}$ of the manganese oxide of the Examples and the Comparative Examples were calculated from the peak intensity ratios (area ratios), respectively. Table 1 showed the results.

The XPS measurement was performed under the following conditions.

Measurement temperature: room temperature
Excitation beam source: $MgK\alpha$ (12 kV, 50 mA)
Sample holder installation angle: 0°

The spectral peaks acquired by XPS were subjected to electrification correction at 284.8 eV using a peak of C1s, and the background correction was performed using the Shirley method. The curve fitting (waveform separation) was performed by the Gauss-Lorentz method, and the $Mn^{3+}$ peaks (641.4 ± 0.5 eV) and the $Mn^{4+}$ peaks (642.6 ± 0.6 eV) were fit. The areas of the $Mn^{3+}$ peaks and the $Mn^{4+}$ peaks obtained by the curve fitting were calculated in the region corresponding to Mn 2p3/2 (636 to 650 eV), and the ratios of the areas of the $Mn^{3+}$ peaks to the areas of the $Mn^{4+}$ peaks were calculated as the values of the $Mn^{3+}/Mn^{4+}$.

Here, the waveform separation was performed in the curve fitting by adjusting the energy value, the half value width, the intensity so as to approximate the observed spectrum. However, the value of the residual sum of squares of the least squares method shown in following expression (a) was defined as 2 or less.

$$x^2 = \frac{1}{N} \sum \left( (I0 - If)^2 / If \right) \qquad \cdots (\text{a})$$

$x^2$: Residual sum of squares
N: Number of fitting points
I0: Observed spectral intensity
If: Intensity of result by fitting calculation

(Measurement of specific surface area)

The nitrogen adsorption isotherm was measured at the temperature of liquid nitrogen (-196°C) using a highly precise gas adsorption measuring device (manufactured by MicrotracBEL Corp.). The specific surface area of the manganese oxide of each of the Examples and the Comparative Examples was calculated based on the BET method. Table 1 showed the results.

(Measurement of X-ray diffraction pattern)

The X-ray diffraction measurement was performed using an X-ray diffraction device for the catalysts of each of the Examples and the Comparative Example, and the diffraction angle of the maximum intensity peak (hereinafter also referred to as a "diffraction angle of the maximum peak") was detected. Table 1 showed the results. The measurement was performed with a SmartLab, manufactured by Rigaku Corporation, under the measurement conditions that X-ray source: Cu-K$\alpha$, X-rays output: 40 kV, 30 mA, scan speed: 2.0 deg/min, step width: 0.02 deg, scan axis: 2θ/θ, and scan range: 5.0 to 90.0 deg.

(Ammonia decomposition evaluation)

Mixed gas of Ar gas, $O_2$ gas, and Ar gas in a volume ratio of 50:20:30 which comprises ammonia at 100 ppm was used as gas to be treated, and was fed to 0.15 g of the deodorizing catalyst of each of the Examples and the Comparative Examples filled in a reaction container at a flow rate of 100 mL/minute while the flow rate was controlled with a flow rate controller (space velocity: 40,000 mlh$^{-1}$g-cat$^{-1}$). Both the temperatures of the deodorizing catalyst and the gas to be treated were adjusted to 80°C. The concentrations of ammonia, dinitrogen monoxide, nitrogen monoxide, and nitrogen dioxide in the gas to be treated before feeding to the deodorizing catalyst and the gas to be treated after feeding to the deodorizing catalyst were measured using an infrared spectrophotometer (FTIR-6000, manufactured by JASCO Corporation). The nitrogen selectivity was calculated based on the following expression (6), and the ammonia decomposition rate was calculated based on the following expression (7). The measurement was continued for at most 10 hours, and the values that reached constant values were defined as measured values. The CAa, CAb, CN1b, CN2b, and C2Nb, shown in the following expression (6), were calculated from the measured values described above.

$$\text{Nitrogen selectivity (\%)} = [\{(CAa - CAb) - (CN1b + CN2b + C2Nb)\}/(CAa - CAb)]*100 \ ... \ (6)$$

CAa: Nitrogen amount of ammonia comprised in gas to be treated before feeding to catalyst (mol/L)
CAb: Nitrogen amount of ammonia comprised in gas to be treated after feeding to catalyst (mol/L)
CN1b: Nitrogen amount of nitrogen monoxide comprised in gas to be treated after feeding to catalyst (mol/L)
CN2b: Nitrogen amount of nitrogen dioxide comprised in gas to be treated after feeding to catalyst (mol/L)
C2Nb: Nitrogen amount of dinitrogen monoxide comprised in gas to be treated after feeding to catalyst (mol/L)

$$\text{Ammonia decomposition rate (\%)} = \{(Ca - Cb)/Ca\}*100 \ ... \ (7)$$

Ca: Concentration of ammonia comprised in gas to be treated before feeding to catalyst (ppm)
Cb: Concentration of ammonia comprised in gas to be treated after feeding to catalyst (ppm)

[Table 1]

| | Diffraction angle of maximum peak ($2\theta$) | Specific surface area (m$^2$/g) | $Mn^{3+}/Mn^{4+}$ | ($Mn^{3+}/Mn^{4+}$) * Specific surface area | Nitrogen selectivity (%) | Ammonia decomposition rate (%) |
|---|---|---|---|---|---|---|
| Example 1 | 37 | 138 | 0.67 | 92 | 100 | 93 |
| Example 2 | 37 | 75 | 0.81 | 61 | 93 | 63 |
| Example 3 | 37 | 241 | 0.75 | 181 | 71 | 65 |
| Example 4 | 37 | 42 | 0.33 | 14 | 98 | 79 |
| Example 5 | 37 | 245 | 0.45 | 110 | 62 | 81 |
| Example 6 | 37 | 175 | 0.75 | 131 | 85 | 90 |
| Example 7 | 37 | 107 | 0.39 | 42 | 100 | 89 |
| Comparative Example 1 | 37 | 222 | 0.93 | 206 | 75 | 55 |
| Comparative Example 2 | 27 | 200 | 0.79 | 158 | 48 | 48 |
| Comparative Example 3 | 27 | 250 | 0.96 | 240 | 42 | 54 |
| Comparative Example 4 | 33 | 42 | 0.54 | 23 | 64 | 49 |
| Comparative Example 5 | 38 | 183 | 0.96 | 176 | 100 | 44 |

As shown in Table 1, the deodorizing catalysts of Examples 1 to 7 all had high ammonia decomposition rates as compared with the deodorizing catalysts of Comparative Examples 1 to 5. It was able to be understood from these results that the deodorizing catalysts of Examples 1 to 7 easily decomposed the malodorous substance even at a low temperature of 100°C or less. As shown in Table 1, the deodorizing catalysts of Examples 1, 2, 4, and 7, which satisfied the above-mentioned expression (4), all had high nitrogen selectivities as compared with Examples 3, 5, and 6, which did not satisfy the above-mentioned expression (4). It was able to be understood from these results that the deodorizing catalysts of Examples 1, 2, 4, and 7, which satisfied the above-mentioned expression (4), was able to decompose the malodorous substance even at a low temperature of 100°C or less, and the nitrogen oxides (harmful substances) were hardly produced in addition even though ammonia was decomposed.

(Hydrogen sulfide decomposition evaluation)

Mixed gas of 20% $O_2$ and 80% $N_2$ comprising 8 ppm hydrogen sulfide was used as gas to be treated, and was fed to 0.01 g of each of the deodorizing catalysts of Example 4 and Example 7 filled in a reaction container at a flow rate of 1.0 L/minute while the flow rate was controlled with a flow rate controller (space velocity: 6,000,000 mlh$^{-1}$g). Both of the temperatures of the deodorizing catalyst and the gas to be treated were adjusted to 25°C. The gas to be treated was continuously fed, and the gas to be treated that permeated the deodorizing catalyst was sampled certain periods of time after the point when the gas to be treated began to be fed as the starting time. The hydrogen sulfide concentration in the sample was measured with a detector tube (No4LB, manufactured by GASTEC CORPORATION). The relative humidity of the gas to be treated was adjusted to 50%, and the hydrogen sulfide removal rate was calculated based on the following expression (8).

```
Hydrogen sulfide decomposition rate (%)= {(Da -

Db)/Da}*100 ... (8)
```

Da: Concentration of hydrogen sulfide comprised in gas to be treated before feeding to deodorizing catalyst (ppm)
Db: Concentration of hydrogen sulfide comprised in gas to be treated after feeding to deodorizing catalyst (ppm)

The hydrogen sulfide decomposition rate in the case of the deodorizing catalyst of Example 4 was 60% 8 minutes after, 50% 29 minutes after, and 30% 139 minutes after, and then became a constant value of around 30%. The hydrogen sulfide removal rate of the deodorizing catalyst of Example 7 was 56% 15 minutes after, 43% 65 minutes after, and 30% 145 minutes after, and then became a constant value of around 30%. It was able to be understood that, as mentioned above, the deodorizing catalysts of Examples 4 and 7 were able to decompose hydrogen sulfide at room temperature (25°C).

**Claims**

1. A deodorizing catalyst for decomposing a malodorous substance, comprising: manganese oxide wherein the manganese oxide satisfies the following expression (1) and the following expression (2), and the manganese oxide has a maximum intensity peak at a diffraction angle (2Θ) of 37 ± 1° in an X-ray diffraction pattern:

$$0 < A \leq 0.90 \; ... \; (1)$$

$$0 < B \leq 250 \; ... \; (2)$$

wherein, in the above-mentioned expression (1), A represents the content ratio of manganese having an oxidation number of 3 ($Mn^{3+}$) to manganese having an oxidation number of 4 ($Mn^{4+}$) ($Mn^{3+}/Mn^{4+}$) in the manganese oxide, and, in the above-mentioned expression (2), B represents a specific surface area of the manganese oxide ($m^2/g$).

2. The deodorizing catalyst according to claim 1, wherein the manganese oxide further satisfies the following expression (3):

$$0 < A \cdot B \leq 200 \; ... \; (3)$$

wherein, in the above-mentioned expression (3), A and B are synonymous with the above.

3. The deodorizing catalyst according to claim 1 or 2, wherein the manganese oxide comprises cations of an alkali metal element.

4. The deodorizing catalyst according to any one of claims 1 to 3,

wherein metal particles are carried on the manganese oxide, and

a metal component comprised in the metal particles is one or more selected from the group consisting of gold, platinum, silver, copper, palladium, rhodium, iridium, ruthenium, osmium, and rhenium.

5. The deodorizing catalyst according to any one of claims 1 to 4, wherein the malodorous substance is one or more substances selected from the group consisting of ammonia, amines, and sulfur compounds.

6. A deodorizing body, comprising: the deodorizing catalyst according to any one of claims 1 to 5 and an adsorbent.

7. A deodorant method, wherein the deodorizing catalyst according to any one of claims 1 to 5 is contacted with fluid to be treated comprising the malodorous substance in the presence of oxygen at a temperature of 20°C or more and 100°C and less.

8. The deodorant method according to claim 7, wherein the deodorizing catalyst is contacted with the fluid to be treated in an environment of a relative humidity of 50% or more.

# EP 4 209 271 A1

## INTERNATIONAL SEARCH REPORT

| International application No. |
|---|
| **PCT/JP2021/032257** |

### A. CLASSIFICATION OF SUBJECT MATTER

*B01J 35/10*(2006.01)i; *F01N 3/08*(2006.01)i; *B01J 23/34*(2006.01)i; *B01J 23/68*(2006.01)i; *A61L 9/00*(2006.01)i; *A61L 9/01*(2006.01)i

FI: B01J23/34 A; B01J35/10 301J; B01J23/68 A; A61L9/00 C; A61L9/01 B; F01N3/08 A

According to International Patent Classification (IPC) or to both national classification and IPC

### B. FIELDS SEARCHED

Minimum documentation searched (classification system followed by classification symbols)

A61L9/00-9/01; A61L9/015-9/04; A61L9/12-9/22; B01J21/00-38/74

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | JP 2013-212459 A (TOYOBO CO LTD) 17 October 2013 (2013-10-17) | 1-3, 5-8 |
| | claims 1, 2, paragraphs [0001], [0012], [0013], [0020], examples 1-12 | |
| Y | | 4 |
| X | JP 2014-108370 A (TOYOBO CO LTD) 12 June 2014 (2014-06-12) | 1-3, 5-8 |
| | claims 1, 2, paragraphs [0001], [0015], [0024], examples 1-8 | |
| Y | | 4 |
| Y | JP 10-137591 A (NIPPON CHEM IND CO LTD) 26 May 1998 (1998-05-26) | 4 |
| | claim 3, paragraph [0010], examples | |
| A | JP 07-228560 A (MITSUI TOATSU CHEM INC) 29 August 1995 (1995-08-29) | 1-8 |
| | fig. 1 | |
| A | JP 2020-022946 A (NITTO DENKO CORP) 13 February 2020 (2020-02-13) | 1-8 |
| | entire text | |

☐ Further documents are listed in the continuation of Box C.  ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **15 October 2021** | **02 November 2021** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

## INTERNATIONAL SEARCH REPORT
### Information on patent family members

International application No.

**PCT/JP2021/032257**

| Patent document cited in search report | | | Publication date (day/month/year) | Patent family member(s) | Publication date (day/month/year) |
|---|---|---|---|---|---|
| JP | 2013-212459 | A | 17 October 2013 | (Family: none) | |
| JP | 2014-108370 | A | 12 June 2014 | (Family: none) | |
| JP | 10-137591 | A | 26 May 1998 | (Family: none) | |
| JP | 07-228560 | A | 29 August 1995 | (Family: none) | |
| JP | 2020-022946 | A | 13 February 2020 | WO 2019/172445 A1 | |

Form PCT/ISA/210 (patent family annex) (January 2015)

**REFERENCES CITED IN THE DESCRIPTION**

**Patent documents cited in the description**

- JP 2009254981 A

- JP 2007216082 A